(19) Europäisches Patentamt
European Patent Office
Office européen des brevets

(11) Veröffentlichungsnummer: **0 291 851 B1**

(12) **EUROPÄISCHE PATENTSCHRIFT**

(45) Veröffentlichungstag der Patentschrift: **28.07.93**

(51) Int. Cl.[5]: **C07D 239/42**, C07D 239/47, C07D 239/52, C07D 239/34, C07D 251/46, C07D 251/14, C07D 251/42, A01N 47/36

(21) Anmeldenummer: **88107591.5**

(22) Anmeldetag: **11.05.88**

Die Akte enthält technische Angaben, die nach dem Eingang der Anmeldung eingereicht wurden und die nicht in dieser Patentschrift enthalten sind.

(54) **Herbizide Sulfonylharnstoffe, Verfahren zu ihrer Herstellung und ihre Verwendung zur Beeinflussung des Pflanzenwachstums.**

(30) Priorität: **19.05.87 DE 3716657**

(43) Veröffentlichungstag der Anmeldung:
**23.11.88 Patentblatt 88/47**

(45) Bekanntmachung des Hinweises auf die Patenterteilung:
**28.07.93 Patentblatt 93/30**

(84) Benannte Vertragsstaaten:
**AT BE CH DE FR GB IT LI NL**

(56) Entgegenhaltungen:
**EP-A- 0 007 687**
**EP-A- 0 161 905**
**EP-A- 0 165 003**
**EP-A- 0 174 212**
**EP-A- 0 202 830**

(73) Patentinhaber: **BASF Aktiengesellschaft**
**Carl-Bosch-Strasse 38**
**W-6700 Ludwigshafen(DE)**

(72) Erfinder: **Hamprecht, Gerhard, Dr.**
**Rote-Turm-Strasse 28**
**W-6940 Weinheim(DE)**
Erfinder: **Hagen, Helmut, Dr.**
**Max-Slevogt-Strasse 17e**
**W-6710 Frankenthal(DE)**
Erfinder: **Liese-Sauer, Thomas, Dr. Glasurit do Brasil LTDa.**
**Av. Angelo Demarchi, 123 Caixa Postal 340**
**09840 Sao Bernardo do Campo-SP(BR)**
Erfinder: **Meyer, Norbert, Dr.**
**Dossenheimer Weg 22**
**W-6802 Ladenburg(DE)**
Erfinder: **Wuerzer, Bruno, Dr.**
**Ruedigerstrasse 13**
**W-6701 Otterstadt(DE)**

EP 0 291 851 B1

Anmerkung: Innerhalb von neun Monaten nach der Bekanntmachung des Hinweises auf die Erteilung des europäischen Patents kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

## Beschreibung

Die Erfindung betrifft neue substituierte 6-Halogen-2-[[(1,3-pyrimidin-2-yl)aminocarbonyl]aminosulfonyl]-benzoesäureester (auch als Sulfonamide, Sulfonylharnstoffe bezeichnet), Verfahren zu ihrer Herstellung, Herbizide und Mittel zur Beeinflussung des Pflanzenwachstums, die die neuen Verbindungen als Wirkstoffe enthalten, sowie ein Verfahren zur Beeinflussung und Bekämpfung von Pflanzenwuchs mit diesen Verbindungen.

In den europäischen Offenlegungsschriften EP-A-0 174 212, EP-A-0 202 830, EP-A-0 161 905, EP-A-0 165 003 und EP-A-0 007 687 werden Sulfonylharnstoff-Derivate mti herbizider Wirkung beschrieben.

Der Erfindung lag die Aufgabe zugrunde, neue Verbindungen mit verbesserten Eigenschaften zu finden.

Überraschend wurde nun gefunden, daß substituierte Sulfonylharnstoffe der Formel

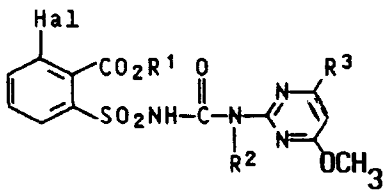

in der

- $R^1$ Wasserstoff oder einen $C_1$-$C_4$-Alkylrest,
- $R^2$ Wasserstoff, Methyl oder Ethyl,
- $R^3$ Fluor oder Chlor,
- Hal Fluor, Chlor oder Brom bedeutet,

oder deren Alkali- oder Erdalkalisalze eine gute Verträglichkeit gegenüber bestimmten Kulturpflanzen, beispielsweise Mais, besitzen.

Neben dem technischen Fortschritt kommen die Endstoffe der Formel I auch einem erheblichen volkswirtschaftlichem Bedürfnis entgegen, nämlich der selektiven Bekämpfung von Hirsearten. So führt M. Hanf in den BASF-Mitteilungen für den Landbau, 1/85, "Ackerunkräuter und Ackerungräser - ihre Verbreitung, Gefährdung und wirtschaftliche Bedeutung" auf S. 23 aus: "Mit Triazinen ließen sich die dikotylen Unkräuter leicht bekämpfen. Die freiwerdenden Flächen wurden aber auch von den, gleich dem Mais gegen diese Mittelgruppe (Triazine) widerstandsfähigen Hirsearten eingenommen. Dies sind vor allem Arten der Gattungen Echinochloa, Digitaria und Setaria. Durch andere spezifische Bekämpfungsmittel und -methoden müssen nun diese Arten wieder zurückgedrängt werden."

Dieses von der Praxis angesprochene Bedürfnis kann von den anmeldungsgemäßen Verbindungen jetzt befriedigt werden.

Bevorzugte Endprodukte der Formel I sind solche, in deren Formel $R^1$ für Wasserstoff oder für $C_1$-$C_4$-Alkylreste also Methyl, Ethyl, n-Propyl, Isopropyl, n-Butyl, Isobutyl, sek-Butyl oder tert.-Butyl steht.

Man erhält die Sulfonylharnstoffe der Formel I z.B. durch (a) Umsetzung von Verbindungen der Formel

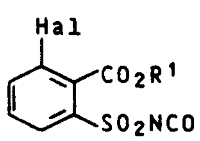

II,

in der $R^1$ und Hal die obengenannte Bedeutung haben, mit einer Verbindung der Formel III

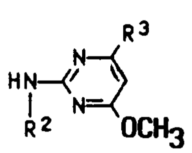

III,

in der $R^2$ und $R^3$ die obengenannten Bedeutungen haben, gegebenenfalls in einem inerten organischen Lösungsmittel bei einer für organische Reaktionen üblichen Temperatur von 0 bis 120 °C, vorzugsweise 10

bis 100 °C. Die Umsetzung kann drucklos oder unter Druck, kontinuierlich oder diskontinuierlich durchgeführt werden.

Verwendet man 6-Fluor-2-isocyanatosulfonyl-benzoesäuremethylester und 2-Amino-4-methoxy-6-methyl-pyrimidin als Ausgangsmaterialien, so kann der Reaktionsablauf durch folgendes Formelschema wiedergegeben werden:

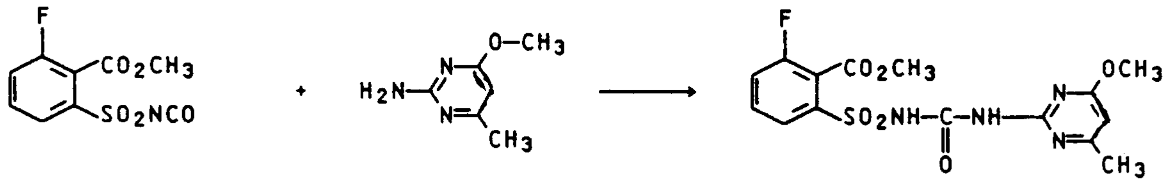

Die Sulfonylharnstoffe der Formel I können auch (b) dadurch erhalten werden, daß man ein Sulfonamid der Formel

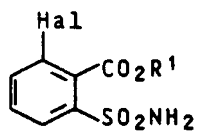

IV,

in der $R^1$ und Hal die obengenannten Bedeutungen haben, mit einem Phenylcarbamat der Formel

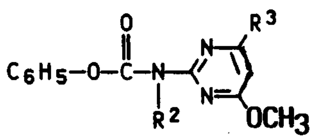

V,

in der $R^2$ und $R^3$ die obengenannten Bedeutungen haben, gegebenenfalls in einem inerten organischen Lösungsmittel und gegebenenfalls in Gegenwart einer tertiären Base bei einer für organische Reaktionen üblichen Temperatur von 0 bis 120 °C, vorzugsweise 10 bis 100 °C umsetzt.

Verwendet man beispielsweise 6-Fluor-2-aminosulfonyl-benzoesäure-methylester und Phenyl-N-(4-methoxy-6-methyl-pyrimidin-2-yl)carbamat als Ausgangsmaterialien, so kann der Reaktionsablauf durch folgendes Formelschema wiedergegeben werden:

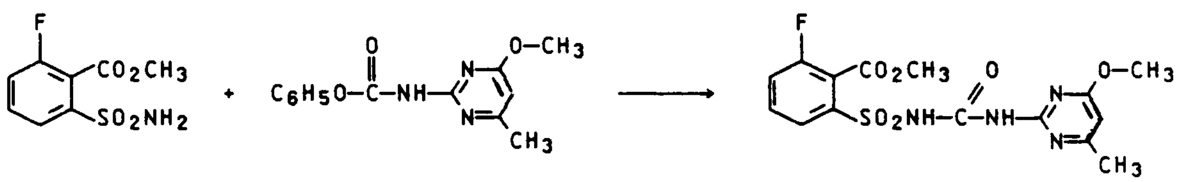

Die Sulfonylharnstoffe der Formel I können auch (c) dadurch erhalten werden, daß man ein Phenylcarbamat der Formel

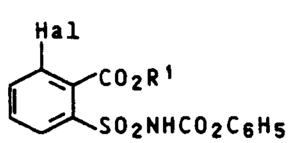

VI,

in der $R^1$ und Hal die vorgenannten Bedeutungen haben, mit einem Amin der Formel

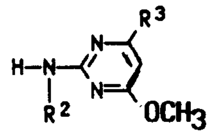

III,

in der $R^2$, und $R^3$ die obengenannten Bedeutungen haben, gegebenenfalls in einem inerten organischen Lösungsmittel und gegebenenfalls in Gegenwart einer tertiären Base bei einer für organische Reaktionen üblichen Temperatur von 0 bis 120 °C, vorzugsweise 10 bis 100 °C umsetzt.

Verwendet man beispielsweise 6-Fluor-2-(phenoxycarbamoylsulfonyl)benzoesäuremethylester und 2-Amino-4-methoxy-6-methyl-pyrimidin als Ausgangsmaterialien, so kann der Reaktionsablauf durch folgendes Formelschema wiedergegeben werden:

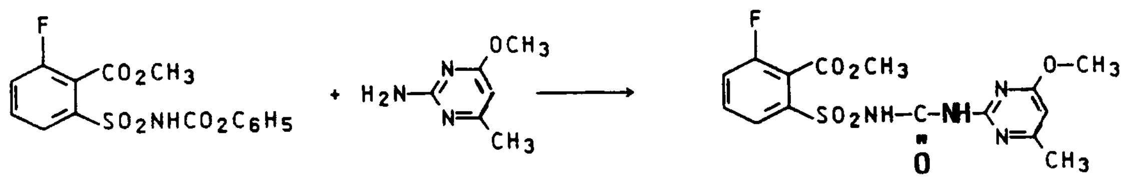

Zweckmäßigerweise verwendet man für die Umsetzungen unter den jeweiligen Reaktionsbedingungen inerte Lösungs- oder Verdünnungsmittel. Als Lösungsmittel kommen beispielsweise Halogenkohlenwasserstoffe, insbesondere Chlorkohlenwasserstoffe, z. B. Tetrachlorethylen, 1,1,2,2-oder 1,1,1,2-Tetrachlorethan, Dichlorpropan, Methylenchlorid, Dichlorbutan, Chloroform, Chlornaphthalin, Dichlornaphthalin, Tetrachlorkohlenstoff, 1,1,1- oder 1,1,2-Trichlorethan, Trichlorethylen, Pentachlorethan, o-, m-, p-Difluorbenzol, 1,2-Dichlorethan, 1,1-Dichlorethan, 1,2-cis-Dichlorethylen, Chlorbenzol, Fluorbenzol, Brombenzol, Jodbenzol, o-, m-, p- Dichlorbenzol, o-, p-, m-Dibrombenzol, o-, m-, p-Chlortoluol, 1,2,4-Trichlorbenzol; Ether, z. B. Ethylpropylether, Methyl-tert.-butylether, n-Butylethylether, Di-n-butylether, Diisobutylether, Diisoamylether, Diisopropylether, Anisol, Phenetol, Cyclohexylmethylether, Diethylether, Ethylenglykoldimethylether, Tetrahydrofuran, Dioxan, Thioanisol, $\beta,\beta'$-Dichlordiethylether;
Nitrokohlenwasserstoffe wie Nitromethan, Nitroethan, Nitrobenzol, o-, m-, p-Chlornitrobenzol, o-Nitrotoluol; Nitrile wie Acetonitril, Butyronitril, Isobutyronitril, Benzonitril, m-Chlorbenzonitril; aliphatische oder cycloaliphatische Kohlenwasserstoffe, z. B. Heptan, Pinan, Nonan, o-, m-, p-Cymol, Benzinfraktionen innerhalb eines Siedepunktintervalles von 70 bis 190 °C, Cyclohexan, Methylcyclohexan, Dekalin, Petrolether, Hexan, Ligroin, 2,2,4-Trimethylpentan, 2,2,3-Trimethylpentan, 2,3,3-Trimethylpentan, Octan; Ester, z. B. Ethylacetat, Acetessigester, Isobutylacetat; Amide, z. B. Formamid, Methylformamid, Dimethylformamid; Ketone, z. B. Aceton, Methylethylketon und entsprechende Gemische in Betracht. Zweckmäßig verwendet man das Lösungsmittel in einer Menge von 100 bis 2.000 Gew.%, vorzugsweise von 200 bis 700 Gew.%, bezogen auf die Ausgangsstoffe II, IV oder VI.

Die zur Umsetzung benötigten Verbindungen III oder V werden im allgemeinen in etwa äquimolaren Mengen (mit einem Über- oder Unterschuß von z.B. 0 bis 20 %, bezogen auf die jeweiligen Ausgangsstoffe II, IV oder VI) eingesetzt. Man kann die Ausgangsstoffe II, IV oder VI in einem der vorgenannten Verdünnungsmittel vorlegen und dann den Ausgangsstoff III bei der Umsetzung (a) und (c) bzw. V bei der Umsetzung (b) zugeben.

zweckmäßigerweise wird das Verfahren zur Herstellung der neuen Verbindungen jedoch so durchgeführt, daß man den Ausgangsstoff III bzw. V, gegebenenfalls in einem der vorgenannten Verdünnungsmittel vorlegt und dann den Ausgangsstoff II (bei der Umsetzung a), oder IV (bei der Umsetzung b), bzw. VI (bei der Umsetzung c) zugibt.

Zur Beendigung der Umsetzung rührt man nach der Zugabe der Komponenten noch 20 Minuten bis 24 Stunden bei 0 bis 120 °C, vorzugsweise 10 bis 100 °C, insbesondere 20 bis 80 °C, nach.

Als Reaktionsbeschleuniger kann man bei der Umsetzung (b) oder (c) vorteilhafterweise eine tertiäre Base, z. B. Pyridin, $\alpha,\beta,\gamma$-Picolin, 2,4-, 2,6-Lutidin, 2,4,6-Collidin, p-Dimethylaminopyridin, 1,4-Diaza[2,2,2]-bicyclooctan [DABCO] oder 1,8-Diazabicyclo[5,4,0]undec-7-en in einer Menge von 0.01 bis 1 Mol pro Mol Ausgangsstoff IV oder VI verwenden.

Die Salze der Sulfonylharnstoffe erhält man durch Umsetzung mit einer stöchiometrischen Menge einer wäßrigen Base oder eines Metallalkoholats, gegebenenfalls in einem inerten organischen Lösungsmittel.

Aus dem Reaktionsgemisch der Umsetzungen a-c wird der Endstoff I in üblicher Weise, z. B. nach Abdestillieren von Lösungsmittel oder direkt durch Absaugen isoliert. Der verbleibende Rückstand kann noch mit Wasser bzw. verdünnter Säure zur Entfernung basischer Verunreinigungen gewaschen werden. Man kann jedoch auch den Rückstand in einem mit Wasser nicht mischbaren Lösungsmittel lösen und wie beschrieben waschen. Die gewünschten Endstoffe fallen hierbei in reiner Form an, gegebenenfalls können sie durch Umkristallisation oder Chromatographie gereinigt werden.

Verbindungen der Formel I, in der $R^1$ Wasserstoff bedeutet, erhält man durch Hydrolyse von Estern der Formel I, in der $R^1$ einen $C_1$-$C_4$-Alkylrest bedeutet. Die Hydrolyse wird mit mindestens der doppelten Menge einer Base wie Natrium- oder Kaliumhydroxid, zweckmäßig in einem Lösungsmittelgemisch mit der 2 bis 8 fachen Menge Methanol und der 10 bis 40 fachen Menge Wasser, bezogen auf das Gewicht des Esters der Formel II, bei 30 bis 80 °C während 1 bis 20 Stunden durchgeführt. Durch Ansäuern fällt man die Sulfonamidcarbonsäuren der Formel I aus.

Die als Ausgangsstoffe der Formel IV benötigten Sulfonamide lassen sich durch Meerwein-Reaktion von 6-Halogen-anthranilsäureestern und anschließende Umsetzung mit Ammoniak herstellen.

Herstellbeispiel für das Vorprodukt 6-Chlor-2-aminosulfonyl-benzoesäuremethylester

93 g 4-Chlor-1,2-benzisothiazol-3-on-1,1-dioxid wurden in 0.8 l Methanol suspendiert und unter Begasen mit Chlorwasserstoff während 3 Stunden unter Rückfluß gerührt. Nach dem Abkühlen auf 20 °C, Absaugen und Trocknen wurden 60 g der Titelverbindung vom Fp. 152 - 153 °C erhalten. Durch Einengen des Filtrats im Vakuum und Verreiben des Rückstands mit Methyl-tert.-butylether, Absaugen und Trocknen erhielt man 42 g einer zweiten Fraktion vom Fp 144 bis 149 °C.

Analog können etwa erhalten werden (bzw. wurden hergestellt, soweit physikalische Angaben beigefügt sind) Verbindungen der allgemeinen Struktur

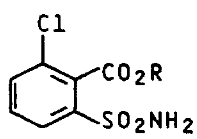

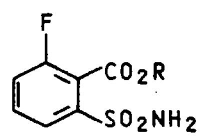

| R | Fp (°C) | Fp (°C) |
|---|---|---|
| $C_2H_5$ | 97 - 101 | 129 - 131 |
| $n-C_3H_7$ | 111 - 113 | 104 - 107 |
| $i-C_3H_7$ | 145 - 147 | 84 - 87 |
| $n-C_4H_9$ | | |
| $i-C_4H_9$ | | |
| sek. $C_4H_9$ | | |

Herstellbeispiel für das Vorprodukt 6-Chlor-2-(isocyanatosulfonyl)benzoesäuremethylester

100 g 6-Chlor-2-aminosulfonyl-benzoesäuremethylester wurden in 300 ml 1,2-Dichlorethan suspendiert, unter Rühren mit 123 g Thionylchlorid versetzt und langsam bis zum Rückfluß erhitzt. Nach 5 Stunden Rühren unter Rückfluß wurde auf 55 °C abgekühlt, 1.5 ml Pyridin zugegeben und unter Einleiten von Phosgen wieder zum Rückfluß erhitzt. Nach 4 Stunden Begasen wurde das Reaktionsgemisch im Vakuum eingeengt und mit Stickstoff belüftet. Das verbliebene Öl (105 g) wurde ohne weitere Reinigung für die Folgestufen eingesetzt.

Herstellbeispiel für das Vorprodukt 6-Fluor-2-chlorsulfonyl-benzoesäuremethylester

108 g 6-Fluoranthranilsäuremethylester und 45 g Natriumnitrit in 106 ml Wasser wurden unter Rühren bei 5 °C über 2 Zulaufvorrichtungen so zu 250 ml konz. Salzsäure innerhalb 1 Stunde gegeben, daß die

Esterkomponente im Überschuß vorlag. Nach 20 Minuten Rühren bei 5 bis 8 °C wurde die Reaktionsmischung in eine vorbereitete Lösung von 53 g Schwefeldioxid, 1.7 g Kupfer(II)chlorid in wenig Wasser in 200 ml 1,2-Dichlorethan in einem Guß gegeben und 10 Minuten nachgerührt. Es wurde langsam auf 50 °C erwärmt und unter Einleiten von 46 g Schwefeldioxid 1 1/2 Stunden gerührt. Dann wurde auf 20 °C abgekühlt und während 20 Minuten 5.5 g Chlor unter Rühren eingeleitet. Es wurde noch 20 Minuten nachgerührt und die organische Phase abgetrennt. Nach dem Waschen mit Wasser, Trocknen über Magnesiumsulfat erhielt man 105 g der Titelverbindung als ein bräunliches Öl.

Herstellbeispiel für das Vorprodukt 6-Fluor-2-aminosulfonyl-benzoesäuremethylester

42.5 g Ammoniak wurden bei 20 bis 28 °C unter Rühren in eine Mischung von 252.5 g 6-Fluor-2-chlorsulfonyl-benzoesäuremethylester in 700 ml wasserfreiem Tetrahydrofuran eingegast. Nach einer Stunde Rühren bei 25 °C wurde der ausgefallene Niederschlag abgesaugt, in Wasser gelöst und einmal mit Essigester extrahiert. Beim Ansäuern der wäßrigen Phase mit konz. Salzsäure erhielt man 8.8 g 4-Fluor-1,2-benzisothiazol-3-on-1,1-dioxid vom Fp. 210 bis 212 °C.

Das Tetrahydrofuranfiltrat wurde eingeengt, mit Wasser gewaschen, in Essigester aufgenommen, nochmals mit Wasser gewaschen, abgesaugt und getrocknet. Man erhielt 186 g der Titelverbindung von Fp. 155 bis 159 °C.

4-Fluor-1,2-benzisothiazol-3-on-1,1-dioxid

100 g 6-Fluor-2-aminosulfonyl-benzoesäuremethylester wurden in 430 ml 1n Natronlauge eingetragen, auf 40 °C erwärmt und 5 Minuten nachgerührt.

Anschließend wurde mit konz. Salzsäure angesäuert und 20 Minuten nachgerührt. Nach dem Absaugen, Waschen mit Wasser und Trocknen erhielt man 78.4 g der Titelverbindung vom Fp. 210 bis 212 °C.

Beispiel 1

6-Chlor-2-[[(4,6-dimethyl-1,3-pyrimidin-2-yl)aminocarbonyl]aminosulfonyl]benzoesäuremethylester

15 g 6-Chlor-2-(isocyanatosulfonyl)benzoesäuremethylester in 100 ml abs. Acetonitril wurden bei Raumtemperatur innerhalb 10 Minuten unter Rühren und Stickstoff zu einer Suspension von 6.7 g 2-Amino-4.6-dimethylpyrimidin in 50 ml abs. Acetonitril gegeben, wobei eine Temperaturerhöhung von 6 °C eintrat. Nach 5 Stunden Rühren bei 25 °C wurde der ausgefallene Niederschlag abgesaugt, mit 1n Salzsäure und mit Methanol gewaschen und getrocknet. Man erhielt 14.2 g der Titelverbindung vom Fp. 215 bis 217 °C.

Beispiel 2

6-Fluor-2-[[(4,6-dimethoxy-1,3,5-triazin-2-yl)aminocarbonyl]aminosulfonyl]benzoesäure-$\beta$-methoxyethylester

7,1 g 6-Fluor-2-isocyanatosulfonylbenzoesäure-$\beta$-methoxyethylester in 20 ml Methylenchlorid wurden bei 25 °C unter Rühren und Stickstoff innerhalb 10 Minuten zu einer Mischung von 4 g 2-Amino-4,6-dimethoxy-1,3,5-triazin in 40 ml DMF gegeben, wobei sich die Temperatur bis auf 32 °C erhöhte. Nach 14 Stunden Rühren bei 25 °C wurde im Vakuum das Lösungsmittel entfernt und der Rückstand mit Ether und zweimal mit 3 n HCl verrührt. Anschließend wurde in Aceton aufgenommen, vom Unlöslichen abgetrennt und das Filtrat zur Trockne eingeengt. Man erhielt 7 g der Titelverbindung vom Fp. 179 bis 182 °C.

Beispiel 3

6-Chlor-2-[[(4,6-dimethoxy-1,3,5-triazin-2-yl)aminocarbonyl]aminosulfonyl]benzoesäureisopropylester-natriumsalz

4,6 g 6-Chlor-2[[(4,6-dimethoxy-1,3,5-triazin-2-yl)aminocarbonyl]aminosulfonyl]benzoesäureisopropylester wurden in 100 ml Methanol suspendiert, bei 25 °C mit 1,8 g 30 %igem Natriummethylat versetzt und 5 Minuten auf 5 °C bis zur klaren Lösung erwärmt. Nach dem Einengen in Vakuum erhielt man 4,7 g der Titelverbindung vom Fp. 185 °C Zers.

Die folgenden erfindungsgemäßen Verbindungen wurden ensprechend den vorstehenden nicht erfindungsgemäßen Beispielen 1 bis 3 hergestellt (in diesem Falle sind physikalische Angaben beigefügt) oder

können analog hergestellt werden.

Tabelle 1

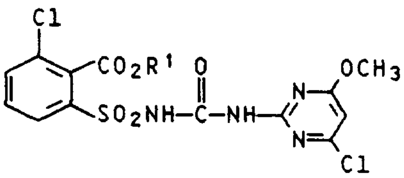

| Beispiel | $R^1$ | Fp (°C) |
|---|---|---|
| 4 | $CH_3$ | 213 Zers. |
| 5 | $C_2H_5$ | 179-182 |
| 6 | $n-C_3H_7$ | 181-184 |
| 7 | $i-C_3H_7$ | 210-212 |
| 8 | $n-C_4H_9$ | |
| 9 | $i-C_4H_9$ | |
| 10 | $sec-C_4H_9$ | |

Tabelle 2

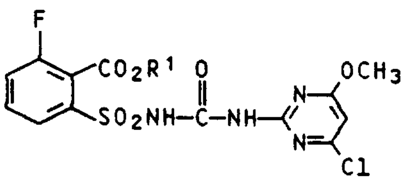

| Beispiel | $R^1$ | Fp (°C) |
|---|---|---|
| 11 | $CH_3$ | 215-217 |
| 12 | $C_2H_5$ | 210-213 |
| 13 | $n-C_3H_7$ | 195-198 |
| 14 | $i-C_3H_7$ | |
| 15 | $n-C_4H_9$ | |
| 16 | $i-C_4H_9$ | |
| 17 | $sec-C_4H_9$ | |

Tabelle 3

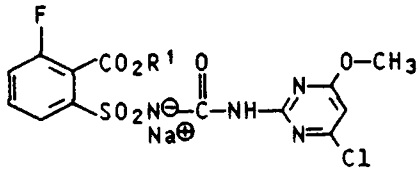

| Beispiel | $R^1$ | Fp (°C) |
|---|---|---|
| 18 | $CH_3$ | >300 |
| 19 | $C_2H_5$ | >300 |
| 20 | $n-C_3H_7$ | >300 |
| 21 | $i-C_3H_7$ | |
| 22 | $n-C_4H_9$ | |
| 23 | $i-C_4H_9$ | |
| 24 | $sec.-C_4H_9$ | |

Tabelle 4

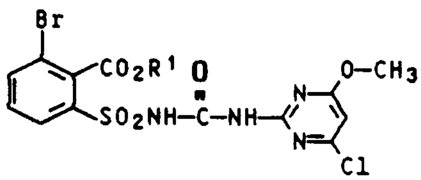

| Beispiel | $R^1$ | Fp (°C) |
|---|---|---|
| 25 | $CH_3$ | 225-228 |
| 26 | $C_2H_5$ | |
| 27 | $n-C_3H_7$ | |

Die Applikation der herbiziden Mittel bzw. der Wirkstoffe kann im Vorauflauf- oder im Nachauflaufverfahren erfolgen. Sind die Wirkstoffe für gewisse Kulturpflanzen weniger verträglich, so können Ausbringungstechniken angewandt werden, bei welchen die herbiziden Mittel mit Hilfe der Spritzgeräte so gespritzt werden, daß die Blätter der empfindlichen Kulturpflanzen nach Möglichkeit nicht getroffen werden, während die Wirkstoffe auf die Blätter darunter wachsender unerwünschter Pflanzen oder die unbedeckte Bodenfläche gelangen (post-directed, lay-by).

Die Aufwandmengen an Wirkstoff betragen je nach Bekämpfungsziel, Jahreszeit, Zielpflanzen und Wachstumsstadien z.B. 0,001 bis 1,0, vorzugsweise 0,005 bis 0,5 kg/ha.

Die herbizide Wirkung der Sulfonamide der Formel I auf das Wachstum der Testpflanzen wird durch folgende Gewächshausversuche gezeigt.

Als Kulturgefäße dienen Plastikblumentöpfe mit 300 $cm^3$ Inhalt und lehmigem Sand mit etwa 3,0 % Humus als Substrat.

Die Samen der Testpflanzen werden nach Arten getrennt flach eingesät. Unmittelbar danach werden bei Vorauflaufanwendungen die Wirkstoffe auf die Erdoberfläche aufgebracht. Sie werden hierzu in Wasser als Verteilungsmittel suspendiert oder emulgiert und mittels fein verteilender Düsen gespritzt.

Die Beispielmengen betragen dabei 0,015, 0,03, 0,04, 0,06 kg Wirkstoff/ha. Nach dem Aufbringen der Wirkstoffe werden die Gefäße leicht beregnet, um Keimung und Wachstum in Gang zu bringen. Danach werden die Gefäße mit durchsichtigen Plastikhauben abgedeckt, bis die Pflanzen angewachsen sind. Diese Abdeckung bewirkt ein gleichmäßiges Keimen der Testpflanzewn, sofern diese nicht durch die Wirkstoffe beeinträchtigt werden.

Für die Nachbehandlung werden die Testpflanzen je nach Wuchsform erst bis zu einer Wuchshöhe von 3 bis 15 cm angezogen und danach behandelt. Zur Nachauflaufbehandlung werden entweder direkt gesäte und in den gleichen Gefäßen aufgewachsene Pflanzen ausgewählt, oder aber sie werden erst als Keimpflanzen in Saatschalen getrennt angezogen und einige Tage vor der Behandlung in die Versuchsgefäße verpflanzt. Eine Abdeckung unterbleibt bei der Nachauflaufbehandlung. Die Aufwandmengen für die Nachauflaufbehandlung betragen beispielsweise 0,015, 0,03, 0,06, 0,125 kg Wirkstoff/ha.

Die Versuchsgefäße werden im Gewächshaus aufgestellt, wobei für wärmeliebende Arten wärmere Bereiche (20 bis 35 °C) und für solche gemäßigter Klimate 10 bis 20 °C bevorzugt werden. Die Versuchsperiode erstreckt sich über 2 bis 4 Wochen. Wahrend dieser Zeit werden die Pflanzen gepflegt und ihre Reaktion auf die einzelnen Behandlungen wird ausgewertet. Bewertet wird nach einer Skala von 0 bis 100. Dabei bedeutet 100 kein Aufgang der Pflanzen bzw. völlige Zerstörung zumindest der oberirdischen Teile und 0 keine Schädigung oder normaler Wachstumsverlauf.

Man schätzt dabei visuell den Einfluß der Behandlung im Vergleich zur unbehandelten Kontrolle.

Die in den Gewächshausversuchen verwendeten Pflanzen sind Unkräuter und Nutzpflanzen z.B. der folgenden Arten:

| Abkürzung | Latein. Name | Englischer Name | Deutscher Name |
|---|---|---|---|
| AMARE | Amaranthus retroflexus | redroot pigweed | Zurückgekrümmter Fuchsschwanz |
| CHEAL | Chenopodium album | lambsquarters (goosefoot) | Weißer Gänsefuß |
| CHYCO | Chrysanthemum coronarium | crown daisy | Kronenwucherblume |
| CYPES | Cyperus esculentus | yellow nutsedge | Erdmandel |
| CYPIR | Cyperus iria | rice flatsedge | - |
| ECHCG | Echinochloa cruss-galli | barnyardgrass | Hühnerhirse |
| IPOSS | Ipomoea spp. | morningglory | Prunkwindenarten |
| LAMAM | Lamium amplexicaule | henbit | Stengelumfassende Taubnessel |
| LOLMU | Lolium multiflorum | annual raygrass | Ital. Raygras |
| POLAV | Polygonum aviculare | prostrate knotweed | Vogelknöterich |
| SOLNI | Solanum nigrum | black nightshade | Schwarzer Nachtschatten |
| STEME | Stellaria media | chickweed | Vogelsternmiere |
| TRZAS | Triticum aestivum | wheat | Weizen |
| ZEAMX | Zea mays | indian corn | Mais |

Zur Bekämpfung von gasartigen und breitblättrigen Pflanzen eignet sich die Verbindung Nr. 11 bei Nachauflaufanwendung mit 0,125 kg a.S./ha und bei Vorauflaufanwendung mit 0.04 kg a.S./ha im Gewächshaus. Mais als Kulturpflanze wird dabei nicht geschädigt.

In Anbetracht der Vielseitigkeit der Applikationsmethoden können die erfindungsgemäßen Verbindungen bzw. die enthaltende Mittel noch in einer weiteren Zahl von Kulturpflanzen zur Beseitigung unerwünschter Pflanzen eingesetzt werden. In Betracht kommen beispielsweise folgende Kulturen:

| Botanischer Name | Deutscher Name |
|---|---|
| Allium cepa | Küchenzwiebel |
| Ananas comosus | Ananas |
| Arachis hypogaea | Erdnuß |
| Asparagus officinalis | Spargel |
| Brassica napus var. napus | Raps |
| Brassica napus var. napobrassica | Kohlrübe |
| Brassica napus var. rapa | Weiße Rübe |
| Camellia sinensis | Teestrauch |
| Carthamus tinctorius | Saflor - Färberdistel |
| Carya illinoinensis | Pekannußbaum |

| Botanischer Name | Deutscher Name |
|---|---|
| Citrus limon | Zitrone |
| Citrus maxima | Pampelmuse |
| Citrus reticulata | Mandarine |
| Citrus sinensis | Apfelsine, Orange |
| Coffea arabica (Coffea canephora, Coffea liberica) | Kaffee |
| Cynodon dactylon | Bermudagras |
| Elaeis guineensis | Ölpalme |
| Fragaria vesca | Erdbeere |
| Glycine max | Sojabohne |
| Gossypium hirsutum (Gossypium arboreum, Gossypium herbaceum, Gossypium vitifolium) | Baumwolle |
| Helianthus annuus | Sonnenblume |
| Helianthus tuberosus | Topinambur |
| Hevea brasiliensis | Parakautschukbaum |
| Hordeum vulgare | Gerste |
| Humulus lupulus | Hopfen |
| Ipomoea batatas | Süßkartoffeln |
| Juglans regia | Walnußbaum |
| Linum usitatissimum | Faserlein |
| Lycopersicon lycopersicum | Tomate |
| Malus spp. | Apfel |
| Manihot esculenta | Maniok |
| Medicago sativa | Luzerne |
| Mentha piperita | Pfefferminze |
| Musa spp. | Obst- und Mehlbanane |
| Nicotiana tabacum (N. rustica) | Tabak |
| Olea europaea | Ölbaum |
| Oryza sativa | Reis |
| Panicum miliaceum | Rispenhirse |
| Phaseolus lunatus | Mondbohne |
| Phaseolus mungo | Erdbohne |
| Phaseolus vulgaris | Buschbohnen |
| Pennisetum glaucum | Perl- oder Rohrkolbenhirse |
| Petroselinum crispum spp. tuberosum | Wurzelpetersilie |

| Botanischer Name | Deutscher Name |
|---|---|
| Picea abies | Rotfichte |
| Abies alba | Weißtanne |
| Pinus spp. | Kiefer |
| Pisum sativum | Gartenerbse |
| Prunus avium | Süßkirsche |
| Prunus domestica | Pflaume |
| Prunus dulcis | Mandelbaum |
| Prunus persica | Pfirsich |
| Pyrus communis | Birne |
| Ribes sylvestre | Rote Johannisbeere |
| Ribes uva-crispa | Stachelbeere |
| Ricinus communis | Rizinus |
| Saccharum officinarum | Zuckerrohr |
| Secale cereale | Roggen |
| Sesamum indicum | Sesam |
| Solanum tuberosum | Kartoffel |
| Sorghum bicolor (S. vulgare) | Mohrenhirse |
| Sorghum dochna | Zuckerhirse |
| Spinacia oleracea | Spinat |
| Theobroma cacao | Kakaobaum |
| Trifolium pratense | Rotklee |
| Triticum aestivum | Weizen |
| Vaccinium corymbosum | Kulturheidelbeere |
| Vaccinium vitis-idaea | Preißelbeere |
| Vicia faba | Pferdebohnen |
| Vigna sinensis (V. unguiculata) | Kuhbohne |
| Vitis vinifera | Weinrebe |
| Zea mays | Mais |

Zur Verbreiterung des Wirkungsspektrums und zur Erzielung synergistischer Effekte können die erfindungsgemäßen Wirkstoffe der Formel I sowohl unter sich als auch mit zahlreichen Vertretern anderer herbizider oder wachstumsregulierender Wirkstoffgruppen gemischt und gemeinsam ausgebracht werden. Beispielsweise kommen als Mischungspartner Diazine, 4H-3,1-Benzoxazinderivate, Benzothiadiazinone, 2,6-Dinitroaniline, N-Phenylcarbamate Thiolcarbamate, Halogencarbonsäuren, Triazine, Amide, Harnstoffe, Diphenylether, Triazinone, Uracile, Benzofuranderivate, Chinolincarbonsäuren, Cyclohexenonverbindungen und andere Wirkstoffe in Betracht.

Außerdem kann es von Nutzen sein, die Sulfonylharnstoffe der Formel I allein oder in Kombination mit anderen Herbiziden auch noch mit weiteren Pflanzenschutzmitteln gemischt gemeinsam auszubringen, beispielsweise mit Mitteln zur Bekämpfung von Schädlingen oder phytopathogenen Pilzen bzw. Bakterien. Von Interesse ist ferner die Mischbarkeit mit Mineralsalzlösungen, welche zur Behebung von Ernährungs- und Spurenelementmängeln eingesetzt werden. Es können auch nichtphytotoxische Öle und Ölkonzentrate zugesetzt werden.

Tabelle I

Vergleichsversuch zur Bekämpfung unerwünschten Pflanzenwachstums im Mais bei Nachauflaufbehandlung im Gewächshaus mit der erfindungsgemäßen Verbindung (A, Beispiel **11**)

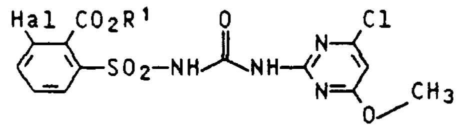

im Vergleich mit dem aus der US-PS 4 547 215 bekannten Wirkstoff Chlorimuron-ethyl (B)

| | | | | Testpflanzen und Schädigung (%) | | | | |
|---|---|---|---|---|---|---|---|---|
| | $R^1$ | Hal | kg a.S./ha | ZEAMX | ECHCG | LOLMU | AMARE | CHYCO |
| A | $CH_3$ | F | 0,125 | 10 | 98 | 98 | 100 | 100 |
| B | $C_2H_5$ | H | 0,03 | 50 | 95 | 50 | 100 | 100 |

Bsp.-Nr. 11 eignet sich mit 0,125 kg a.S./ha im Nachauflaufverfahren zur Bekämpfung grasartiger und breitblättriger unerwünschter Pflanzen ohne die Kulturpflanze Mais nennenswert zu schädigen. Das Vergleichsmittel Chorimuron-ethyl erfaßt zwar breitblättrige Pflanzen, aber schon bei 0,03 kg a.S./ha wird der Mais massiv geschädigt ohne daß grasartige unerwünschte Pflanzen hinreichend bekämpft werden. Der Vergleichsversuch zeigt deutliche Vorteile für Bsp.-Nr. 11.

Tabelle II

Herbizide Aktivität gegen grasartige und breitblättrige Beispielspflanzen und Verträglichkeit für Mais bei Vorauflaufanwendung von 0,04 kg a.S./ha im Gewächshaus der erfindungsgemäßen Verbindung **11**

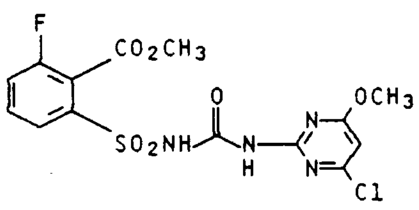

Testpflanzen und Schädigung (%)

| | |
|---|---|
| ZEAMX | 0 |
| ECHCG | 90 |
| CHYCO | 98 |
| SOLNI | 95 |

## Patentansprüche

1. Substituierte Sulfonylharnstoffe der Formel I

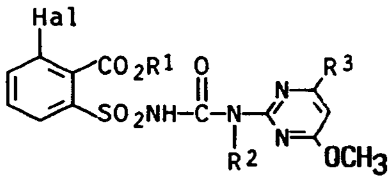

I

in der
$R^1$ Wasserstoff oder einen $C_1$-$C_4$-Alkylrest,
$R^2$ Wasserstoff, Methyl oder Ethyl,
$R^3$ Fluor oder Chlor,
Hal Fluor, Chlor oder Brom bedeutet,
oder deren Alkali- oder Erdalkalisalze.

2. Sulfonylharnstoffe der Formel I gemäß Anspruch 1, dadurch gekennzeichnet, daß $R^1$ einen $C_1$-$C_4$-Alkylrest und Hal Fluor oder Chlor bedeuten.

3. Verfahren zur Herstellung von Sulfonylharnstoffen der Formel I gemäß Anspruch 1, unter Ausnahme der Bedeutung von $R^1$ = Wasserstoff, dadurch gekennzeichnet, daß man einen entsprechenden 6-Halogen-2-(isocyanatosulfonyl)benzoesäureester der Formel II

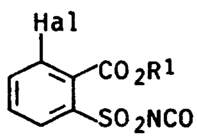

II,

in der $R^1$ die vorgenannte Bedeutung außer Wasserstoff besitzt und Hal für Fluor, Chlor oder Brom steht, mit ungefähr stöchiometrischen Mengen eines entsprechenden Amins der Formel III

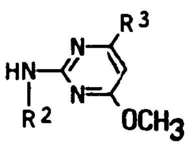

III,

bei einer für organische Reaktionen üblichen Temperatur im Bereich von 0 bis 120 °C umsetzt.

4. Verfahren zur Herstellung von Sulfonylharnstoffen der Formel I gemäß Anspruch 3, dadurch gekennzeichnet, daß man ein entsprechendes Sulfonamid der Formel IV

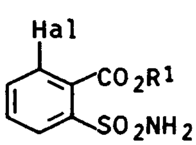

IV,

mit ungefähr stöchiometrischen Mengen eines entsprechenden Phenylcarbamats der Formel V

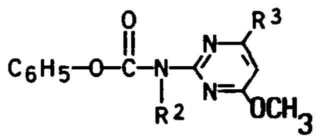

V,

bei einer für organische Reaktionen üblichen Temperatur im Bereich von 0 bis 120 °C umsetzt.

**5.** Verfahren zur Herstellung von Sulfonylharnstoffen der Formel I gemäß Anspruch 1, dadurch gekennzeichnet, daß man ein entsprechendes Phenylcarbamat der Formel VI

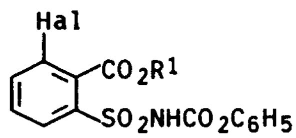

VI,

mit ungefähr stöchiometrischen Mengen eines entsprechenden Amins der Formel III

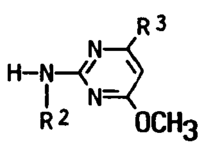

III,

bei einer für organische Reaktionen üblichen Temperatur im Bereich von 0 bis 120 °C umsetzt.

**6.** Herbizides Mittel, enthaltend einen Sulfonylharnstoff der Formel I gemäß Anspruch 1 oder eines seiner Salze neben hierfür üblichen Trägerstoffen.

**7.** Verfahren zur Bekämpfung unerwünschten Pflanzenwuchses, dadurch gekennzeichnet, daß man einen Sulfonylharnstoff der Formel I gemäß Anspruch 1 oder eines seiner Salze auf die Pflanzen und/oder ihren Lebensraum einwirken läßt.

**8.** Mittel zur Beeinflussung des Pflanzenwuchses, enthaltend neben inerten Zusatzstoffen einen Sulfonylharnstoff der Formel I gemäß Anspruch 1 oder eines seiner Salze.

**9.** Verfahren zur Bekämpfung unerwünschten Pflanzenwuchses in Getreidekulturen, dadurch gekennzeichnet, daß man 6-Fluor-2[[(4-chlor-6-methoxy-1,3-pyrimidin-2-yl)amincarbonyl]aminosulfonyl]-benzoesäuremethylester oder dessen Natriumsalz verwendet.

**10.** Substituierte Sulfonylharnstoffe der Formel I gemäß Anspruch 1, in der $R^1$ $C_1$-$C_4$-Alkyl, $R^2$ Wasserstoff, $R^3$ Chlor und Hal Fluor oder Chlor bedeuten oder deren Alkalisalze.

**11.** 6-Chlor-2[[(4-chlor-6-methoxy-1,3-pyrimidin-2-yl)aminocarbonyl]aminosulfonyl]benzoesäureethylester.

**12.** 6-Fluor-2[[(4-chlor-6-methoxy-1,3-pyrimidin-2-yl)aminocarbonyl]aminosulfonyl]benzoesäureethylester.

**Claims**

**1.** A substituted sulfonylurea of the formula I where

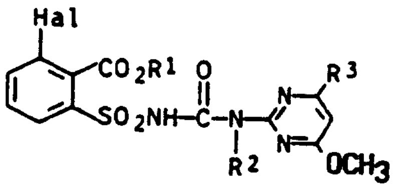

I

R1 is hydrogen or $C_1$-$C_4$-alkyl,
$R^2$ is hydrogen, methyl or ethyl,
$R^3$ is fluorine or chlorine,
Hal is fluorine, chlorine or bromine.
or the alkali metal or alkaline metal salts thereof.

**2.** A sulfonylurea of the formula I as claimed in claim 1, wherein $R^1$ is $C_1$-$C_4$-alkyl and Hal is fluorine or chlorine.

**3.** A process for the preparation of sulfonylureas of the formula I as claimed in claim 1, except that $R^1$ is not hydrogen, wherein a corresponding 6-halo-2-(isocyanatosulfonyl)benzoate of the formula II

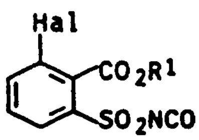

II,

where $R^1$ has the above meanings with the exception of hydrogen, and Hal is fluorine, chlorine or bromine, is reacted with approximately stoichiometric amounts of a corresponding amine of the formula III

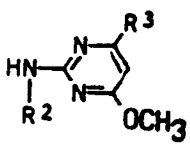

III,

at a temperature customary for organic reactions in the range from 0 to 120 °C.

**4.** A process for the preparation of sulfonylureas of the formula I as claimed in claim 3, wherein a corresponding sulfonamide of the formula IV

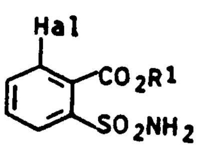

IV,

is reacted with approximately stoichiometric amounts of a corresponding phenyl carbamate of the formula V

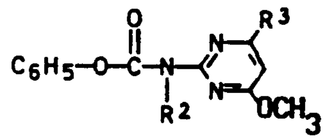 V,

at a temperature customary for organic reactions in the range from 0 to 120 ° C.

**5.** A process for the preparation of sulfonylureas of the formula I as claimed in claim 1, wherein a corresponding phenyl carbamate of the formula VI

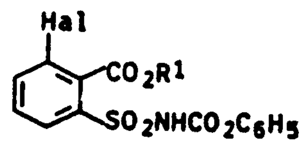 VI,

is reacted with approximately stoichiometric amounts of a corresponding amine of the formula III

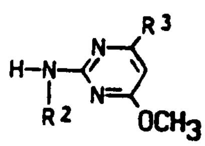 III,

at a temperature customary for organic reactions in the range from 0 to 120 ° C.

**6.** A herbicidal agent containing a sulfonylurea of the formula I as claimed in claim 1, or a salt thereof, in addition to conventional carriers.

**7.** A method for controlling unwanted plant growth, wherein a sulfonylurea of the formula I as claimed in claim 1, or a salt thereof, is allowed to act on the plants and/or their habitat.

**8.** An agent for influencing plant growth, containing in addition to inert additives a sulfonylurea of the formula I as claimed in claim 1, or a salt thereof.

**9.** A method for controlling unwanted plant growth in cereals, wherein methyl 6-fluoro-2[[(4-chloro-6-methoxy-1,3-pyrimidin-2-yl)aminocarbonyl]aminosulfonyl]benzoate or the sodium salt thereof is used.

**10.** A substituted sulfonylurea of the formula I as claimed in claim 1, in which $R^1$ is $C_1$-$C_4$-alkyl, $R^2$ is hydrogen, $R^3$ is chlorine and Hal is fluorine or chlorine or an alkali metal salt thereof.

**11.** Ethyl 6-chloro-2[[(4-chloro-6-methoxy-1,3-pyrimidin-2-yl)aminocarbonyl]aminosulfonyl]benzoate.

**12.** Ethyl 6-fluoro-2[[(4-chloro-6-methoxy-1,3-pyrimidin-2-yl)aminocarbonyl]aminosulfonyl]benzoate.

## Revendications

1. Sulfonylurées substituées de formule I

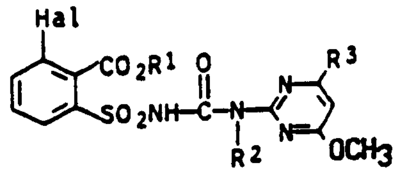

I

dans laquelle
$R^1$ représente l'hydrogène ou un groupe alkyle en C1-C4,
$R^2$ représente l'hydrogène, un groupe méthyle ou éthyle,
$R^3$ représente le fluor ou le chlore,
Hal représente le fluor, le chlore ou le brome,
ou leurs sels alcalins ou alcalino-terreux.

2. Sulfonylurées de formule I selon la revendication 1, caractérisées en ce que $R^1$ représente un groupe alkyle en C1-C4 et Hal le fluor ou le chlore.

3. Procédé de préparation des sulfonylurées de formule I de la revendication 1, à l'exception de celles pour lesquelles $R^1$ représente l'hydrogène, caractérisé en ce que l'on fait réagir à une température usuelle pour les réactions organiques, dans l'intervalle de 0 à 120 degrés C, un ester 6-halogéno-2-(isocyanatosulfonyl)-benzoïque correspondant de formule II

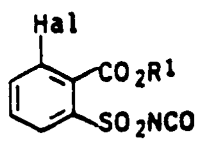

II,

dans laquelle $R^1$ a les significations indiquées ci-dessus à l'exception de l'hydrogène, et Hal représente le fluor, le chlore ou le brome, avec une quantité à peu près stoechiométrique d'une amine correspondante de formule III

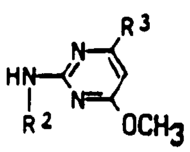

III,

4. Procédé de préparation des sulfonylurées de formule I selon la revendication 3, caractérisé en ce que l'on fait réagir à une température usuelle pour les réactions organiques, dans l'intervalle de 0 à 120 degrés C, un sulfonamide correspondant de formule IV

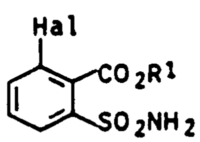

IV,

avec la quantité à peu près stoechiométrique d'un phénylcarbamate correspondant de formule V

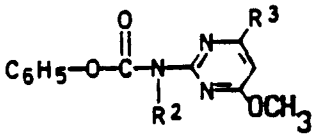

V,

5. Procédé de préparation des sulfonylurées de formule I selon la revendication 1, caractérisé en ce que l'on fait réagir à des températures usuelles pour les réactions organiques, dans l'intervalle de 0 à 120 degrés C, un phénylcarbamate correspondant de formule VI

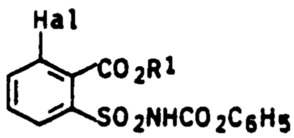

VI,

avec la quantité à peu près stoechiométrique d'une amine correspondante de formule III

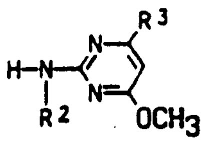

III,

6. Produit herbicide contenant une sulfonylurée de formule I de la revendication 1 ou l'un de ses sels avec des véhicules usuels à cet effet.

7. Procédé pour combattre la croissance de végétaux indésirables, caractérisé en ce que l'on fait agir sur les végétaux et/ou leur habitat une sulfnylurée de formule I de la revendication 1 ou l'un de ses sels.

8. Produit pour agir sur la croissance des végétaux, contenant, avec des additifs inertes, une sulfonylurée de formule I de la revendication 1 ou l'un de ses sels.

9. Procédé pour combattre la croissance de végétaux indésirables dans les cultures de céréales, caractérisé en ce que l'on utilise le 6-fluoro-2-[[(4-chloro-6-méthoxy-1,3-pyrimidine-2-yle)-aminocarbonyl]-aminosulfonyl]-benzoate de méthyle ou son sel de sodium.

10. Sulfonylurées substituées de formule I de la revendication 1 dans laquelle $R^1$ représente un groupe alkyle en C1-C4, $R^2$ l'hydrogène, $R^3$ le chlore et Hal le fluor ou le chlore, ou leurs sels alcalins.

11. Le 6-chloro-2-[[(4-chloro-6-méthoxy-1,3-pyrimidine-2-yl)-aminocarbonyl]-aminosulfonyl)-benzoate d'éthyle.

12. Le 6-fluoro-2-[[(4-chloro-6-méthoxy-1,3-pyrimidine-2-yl)-aminocarbonyl]-aminosulfonyl]-benzoate d'éthyle.